# EUROPÄISCHE PATENTANMELDUNG

(11) **EP 0 597 276 A1**
(43) Veröffentlichungstag der Anmeldung: **18.05.1994**
(21) Anmeldenummer: 93116697.9
(22) Anmeldetag: 15.10.1993
(51) Int. Cl.: C07C 251/24, B01D 61/38, B01D 69/14, B01D 53/22, C01B 13/02

(54) **N,N'-2.2-3.3-Tetramethylethylen-bis(5-NO2-salicyliden-iminato)colbalt(II) und dessen Verwendung als Trägersubstanz für Sauerstoff**

(30) Priorität: 11.11.1992 DE 4238076
(71) Anmelder: FRAUNHOFER-GESELLSCHAFT ZUR FÖRDERUNG DER ANGEWANDTEN FORSCHUNG E.V., D-80636 München (DE)
(72) Erfinder: Bauer, Bernd, Dipl.-Chem., D-70569 Stuttgart (DE)

(57) **Zusammenfassung**

Die Erfindung betrifft eine Cobalt-Verbindung der Formel 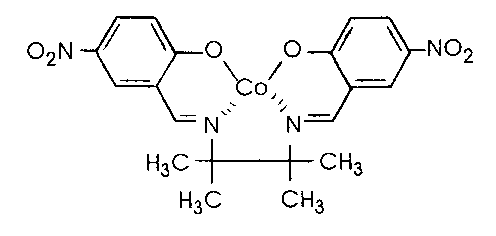
ein Verfahren zu deren Herstellung und deren Verwendung als stoffspezifische Trägersubstanz für Sauerstoff (Sauerstoff-Carrier) bei der Abtrennung von Sauerstoff aus Sauerstoff haltigen Mischungen.

## Beschreibung

Die Erfindung betrifft eine Cobalt-Verbindung der Formel 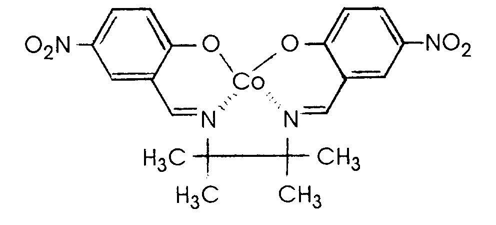
ein Verfahren zu deren Herstellung und deren Verwendung als stoffspezifische Trägersubstanz für Sauerstoff (Sauerstoff-Carrier) bei der Abtrennung von Sauerstoff aus Sauerstoff haltigen Mischungen.

Systematische Bezeichnungen für die erfindungsgemäße Cobalt-Verbindung sind die folgenden:
N,N'-2.2-3.3-Tetramethylethylen-bis(5-NO₂-salicyliden-iminato)cobalt(II)
2.2'-[(1,1,2,2-tetramethyl-1,2-ethandiyl)bis(nitrilomethyliden)]bis[4-nitrophenol]cobalt(II)
([2.2'-[(1,1,2,2-tetramethyl-1,2-ethandiyl)bis(nitrilomethyliden)]bis[4-nitrophenolato]](2-)-N2,N2',O1,O1']-cobalt(II)
Die Summenformel lautet: C₂₀H₂₀CoN₄O₆
Das stoffspezifische Trennen von molekularen Gas- oder Flüssigkeitsgemischen, wie z.B. die Anreicherung von Sauerstoff aus Luft, ist ein zentrales Problem in allen Bereichen der technischen Chemie, der Lebensmittelindustrie, der Pharmazie sowie der Rohstoffgewinnung. Innerhalb der verschiedenen Prozesse, die heute für diese Stofftrennung zur Verfügung stehen, spielen Membrantrennverfahren eine ganz zentrale Rolle. Um mit ihnen Produktionsprozesse wirtschaftlich zu gestalten, ist es erforderlich, daß die eingesetzten Membranen einen möglichst hohen flächenspezifischen Durchsatz und eine möglichst hohe Selektivität aufweisen. Diese Anforderungen sind insbesonders dann schwierig zu erfüllen, wenn Stoffe getrennt werden sollen, die sich nur wenig in ihren physikalischen und chemischen Eigenschaften unterscheiden, wie dies bei Stickstoff und Sauerstoff der Fall ist.

Die meisten heute technisch genutzten Membranen bestehen aus Polymeren und besitzen eine symmetrische oder asymmetrische, mehr oder weniger poröse Struktur. Die stofftrennenden Eigenschaften dieser Membranen werden entweder durch die Größe der Poren oder durch die Löslichkeit und Diffusivität der verschiedenen Komponenten in der Matrix des Membranpolymers bestimmt. Da die geometrischen Abmessungen verschiedener Stoffe, deren Löslichkeit und auch deren Diffusionskoeffizient in einer Polymermatrix oft nicht sehr unterschiedlich sind, ist in vielen Fällen eine Trennung mit herkömmlichen Polymermembranen schwierig und wirtschaftlich häufig uninteressant.

Bei Flüssigmembranen wird anstatt eines Feststoffes eine Flüssigkeit als Membran verwendet, da hier die Diffusionskoeffizienten (D) um Größenordnungen höher sind als in Polymeren (Feststoffen). Damit wird eine Erhöhung des transmembranen Flusses und somit der Permeabilität festgestellt.
- Zum Vergleich:: - Polymer : D = 10⁻¹¹ bis 10⁻¹⁴ m²/s
- Flüssigkeit: D = 10⁻⁹ bis 10⁻¹⁰ m²/s
Jedoch wird die Diffusion sowohl für Sauerstoff als auch für Stickstoff in gleichem Maße erhöht, da diese beiden Gase praktisch den selben Durchmesser besitzen. Weiterhin sagt die Stokes-Einstein-Gleichung voraus, daß beide Gase ungefähr den gleichen Diffusionskoeffizienten in allen Medien besitzen, was auch weitgehend beobachtet wurde. Deshalb kann eine Trennung dieser beiden Gase nur auf deren unterschiedliche Löslichkeiten im Polymer zurückgeführt werden. Zur Trennung dieser beiden Gase müssen die Flüssigkeiten also entweder unterschiedliche Löslichkeiten für diese aufweisen, oder ihnen werden stoffspezifische Trägersubstanzen, sog. Carrier-Substanzen, zugesetzt, welche befähigt sind, eine der Komponenten, z.B. den Sauerstoff, spezifisch binden.

Eine wesentlich bessere Trennung von chemisch und physikalisch sehr ähnlichen Stoffen läßt sich also mit Hilfe stoffspezifischer Trägersubstanzen erreichen, wie sie z.B. in Flüssigmembranen enthalten sind. Bei den Flüssigmembranen unterscheidet man zwischen dem Emulsionstyp und den Matrix gestützten Flüssigmembranen. Emulsionsmembranen bestehen z.B. aus zwei durch Öl-Phasen getrennte Wasserphasen, wobei sich hier die Probleme von Extraktionstechniken, d.h. schlechte Phasentrennung und geringe Stabilität der Phasen, zeigen. Bei den gestützten Flüssigmembranen, welche z.B. für die Gastrennung verwendet werden, wird eine Flüssigkeit aufgrund von Kapillarkräften z.B. in der Porenstruktur einer Polymermatrix fixiert. Solche Flüssigmembranen werden üblicherweise dadurch hergestellt, daß man einen Träger, z.B. eine poröse Folie oder eine Platte mit dem Flüssigmembranmaterial tränkt. Dabei übt die poröse Struktur des Trägers keine trennende Funktion aus, sondern hat nur die Aufgabe, die in ihren Poren eingeschlossene Flüssigkeit mechanisch zu stabilisieren. Die eigentliche Stofftrennung wird von den selektiven Trägersubstanzen in der Flüssigkeit übernommen.

Als poröse Träger eignen sich z.B. Ultrafiltrationsmembranen, die durch ihre geringen Porendurchmesser hohe Kapillarkräfte besitzen und die somit in der Lage sind, die Flüssigkeit auch bei höheren Drücken in der Membran zu halten. Die Flüssigkeit muß dabei bestimmte Voraussetzungen erfüllen. Diese sind:
- geringer Dampfdruck, damit sie sich während des Betriebes nicht verflüchtigt;
- günstige Selektivität bezüglich einer Komponente;
- Benetzbarkeit der Poren des Trägers und chemische Verträglichkeit für den Träger;
- niedrige Viskosität, da die Diffusionskoeffizienten von Gasen umgekehrt proportional zur Viskosität sind.

Der Stofftransport in Flüssigmembranen erfolgt derart, daß die Trägersubstanz in der Membran zirkuliert und an der dem Rohgemisch zugewandten Seite der Membran, der Feed-Seite, einen bestimmten Stoff, z.B. Sauerstoff, selektiv und reversibel aufnimmt, per Diffusion in der Flüssigkeit durch die Membran transportiert und auf der Permeatseite der Membran wieder abgibt. Der unbeladene Carrier diffundiert dann infolge des Konzentrationsgradienten von der Permeat- zur Feed-Seite zurück. Die hohe Selektivität von Flüssigmembranen beruht darauf, daß die Trägersubstanz ausschließlich mit der zu transportierenden Komponente koppelt und alle anderen Stoffe des Gemisches nicht in die Flüssigkeit eindringen und somit durch die Membran nicht permeieren können. Im Falle der Stickstoff/Sauerstoff-Trennung ist eine der beiden Komponenten, z.B. der Stickstoff, nicht befähigt, mit dem Carrier eine Bindung einzugehen.

Flüssigmembranen in Verbindung mit stoffspezifischen Trägersubstanzen zeichnen sich durch sehr hohe Spezifitäten für eine Komponente sowie durch hohe Permeabilitäten aus. Dies gilt besonders für die Trennung von Gasen, wie z.B. für Stickstoff und Sauerstoff, einem Stofftrennproblem von ganz besonderer wirtschaftlicher Bedeutung. Durch die Verwendung von selektiven Trägersubstanzen, z.B. von Kobalt-Salen-Komplexen, in Verbindung mit einer Flüssigmembran lassen sich hier ganz enorme Leistungssteigerungen in der Permeabilität und Selektivität von Polymeren erzielen. Flüssigmembranen mit einem Kobalt-Salen-Komplex zeigen eine ca. 10 bis 20 mal höhere Selektivität und besitzen eine ca. 10 mal höhere Permeabilität als herkömmliche Polymermembranen. Außer den Kobalt-Komplexen sind in der Literatur noch eine Reihe anderer Schwermetallkomplexe beschrieben, die über ausgezeichnete selektive Stofftransporteigenschaften bei der Stickstoff/Sauerstoff-Trennung verfügen. Damit bietet die Flüssigmembrantechnik in Verbindung mit selektiven Trägersubstanzen eine ganz erhebliche technische und wirtschaftliche Verbesserung bei der Nutzung von Membranen zur Trennung von molekularen Gemischen.

Flüssigmembranen sind z.B. aus den folgenden Druckschriften bekannt: EP 0098731 B1, EP 0176986 A2, EP 0186182 A2, EP 0213744 A2, US 3,625,734, US 4,705,544, US 4,174,374 und US 4,710,205.

Aus der EP 0154247 B1 sind Membranen aus Vinylpolymeren bekannt, bei denen Sauerstoff übertragende Gruppen kovalent an die Polymerkette gebunden sind.

Für die Gastrennung, wie z.B. für die Abtrennung von Sauerstoff, werden als stoffspezifische Trägersubstanzen Metall-Chelate, vorwiegend Co-Salen- und Co-Porphin-Verbindungen eingesetzt. Die für die Sauerstoff-Abtrennung verwendeten stoffspezifischen Trägersubstanzen haben die folgenden Anforderungen zu erfüllen, um einen trägergebundenen Sauerstoff-Transport zu gewährleisten:
- Chemische Stabilität der Komplexe, vorallem Oxidationsbeständigkeit;
- Reversibilität der Sauerstoff-Aufnahme;
- Hohe Geschwindigkeit der Sauerstoff-Aufnahme und -Abgabe;
- Mobilität der Komplexe im flüssigen und polymeren Teil der Membran.

Die chemische Stabilität der Co-Komplexe ist vorallem durch deren Oxidationsanfälligkeit limitiert, wobei die Oxidation vornehmlich bei steigenden Temperaturen und höherem Wassergehalt der Lösung erfolgt. Zu einer irreversiblen Bindung des Sauerstoff-Moleküls kommt es z.B. dadurch, daß das Co(II) zum Co(III) oxidiert wird, indem der Co-Komplex in die Superoxo-Form (O₂⁻) übergeht und mit einem zweiten sauerstoffreichen Carrier-Molekül zur Peroxo-Verbindung(O₂²⁻) dimerisiert. Die Peroxo-Verbindung ist dann in der Lage, sich irreversibel in die Oxo-Form (O²⁻) umzuwandeln. Diese Oxidationsanfälligkeit der Sauerstoff-Carrier nach dem Stand der Technik verhindert längere Standzeiten.

In den Co-Salen- und Co-Porphin-Verbindungen, die als Carrier verwendet werden, ist das Zentralatom Co²⁺ von fünf Elektronendonatoren koordiniert. Diese stellen entweder die Liganden oder eine sog. Axialbase dar. Anstelle einer Axialbase kann auch ein geeignetes Lösungsmittel diese Funktion übernehmen. Durch Zugabe einer Axialbase zu einem 4-fach koordinierten Co-Komplex wird der "Bindungszustand" des Co-Atoms zur Koordinationszahl 6 erweitert, wobei die 6. Koordinationsstelle anfangs unbesetzt ist und später bei der Sauerstoffübertragung durch den Sauerstoff eingenommen wird. Dabei wird der molekulare Sauerstoff direkt vom Co²⁺-Atom durch partielle Elektronenübertragung gebunden.

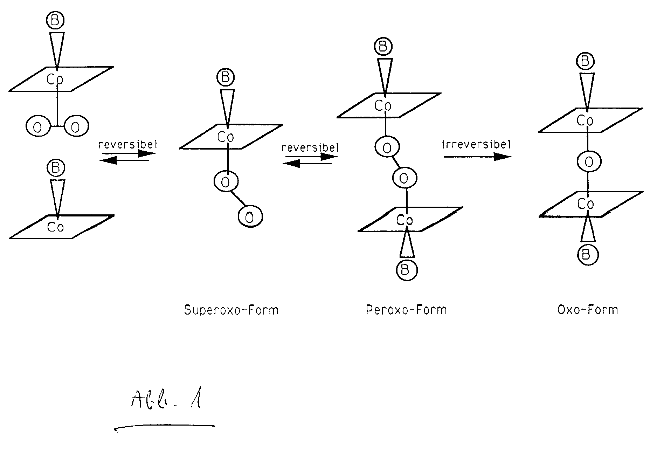

Abbildung 1 zeigt die Bindungsverhältnisse eines mit Sauerstoff beladenen Cobalt-Komplexes sowie dessen irreversible Oxidation über die Superoxo- und Peroxo-Form zur Oxo-Form.

Aufgabe der vorliegenden Erfindung war es nun, eine stoffspezifische Trägersubstanz für Sauerstoff (Sauerstoff-Carrier) zur Verfügung zu stellen, die hinsichtlich der Oxidationsbeständigkeit im Vergleich mit Sauerstoff-Carriern nach dem Stand der Technik stark verbessert ist. Ferner soll dieser Carrier universell anwendbar sein, ohne seine Selektivität zu verlieren, d.h. er soll sowohl in Flüssigmembranen als auch in Fest-Carrier-Membranen eingesetzt werden können.

Gelöst wird diese Aufgabe durch eine Cobalt-Verbindung mit den systematischen Bezeichnungen:
N,N'-2.2-3.3-Tetramethylethylen-bis(5-NO₂-salicyliden-iminato)cobalt(II),
2.2'-[(1,1,2,2-tetramethyl-1,2-ethandiyl)bis(nitrilomethyliden)]bis[4-nitro-phenol]cobalt(II) oder
([2.2'-[(1,1,2,2-tetramethyl-1,2-ethandiyl)bis(nitrilomethyliden)]bis[4-nitro-phenolato]](2-)-N2,N2',O1,O1']-cobalt(II)
mit der Summenformel C₂₀H₂₀CoN₄O₆ und mit der Strukturformel 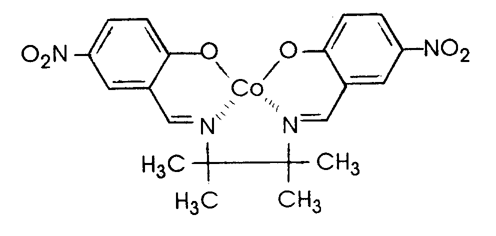
Überraschenderweise wurde nun in dem erfindungsgemäßen Cobalt-Komplex ein Sauerstoff-Carrier gefunden, der eine wesentlich verbesserte Stabilität gegenüber molekularem Sauerstoff zeigt, und der damit wesentlich längere Standzelten bei der Stickstoff/Sauerstoff-Trennung aufweist.

Überraschenderweise wurde damit eine Co-Verbindungen gefunden, bei der das Redox-Potential des Zentralatoms zwar ausreicht, um Elektronen partiell auf Sauerstoff zu übertragen, aber nicht, um den Sauerstoff vollständig zu einem Superoxid oder gar Peroxid zu reduzieren. Damit erfüllt die erfindungsgemäße Verbindung die Anforderungen, die an einen optimalen Sauerstoff-Carrier gestellt werden, nämlich reversible Sauerstoff-Bindung, chemische Stabilität, auch gegenüber Sauerstoff, hohe Geschwindigkeit der Sauerstoff-Aufnahme und -Abgabe, sowie Mobilität des Komplexes im flüssigen und polymeren Teil von Carrier-Membranen.

Es wird angenommen, daß durch den Einbau von zwei Nitro-Gruppen (elektronischer Effekt), jeweils in para-Stellung zum Sauerstoff-Liganden, das Reduktionspotential des Kobalts so verändert wird, daß der Co-Komplex gegenüber Oxidationen stabilisiert ist. Desweiteren wird angenommen, daß durch die Modifizierung der N-N-Brücke (sterischer Effekt) eine zusätzliche Stabilisierung erfolgt.

Im folgenden wird die Synthese der erfindungsgemäßen Cobalt-Verbindung beschrieben.

### 1. Synthese des 2.3-Diamino-2.3-dimethylbutan (2.3-Dimethyl-butandiyldiamin)

In einem 1 l 3-Halskolben, versehen mit Innenthermometer, Rückflußkühler und Magnetrührer werden 17.6 g 2.3-Dinitro-2.3-dimethylbutan in ca. 200 ml konzentrierter Salzsäure unter starkem Rühren auf 50 - 60 °C erhitzt. Zu der Reaktionslösung wird vorsichtig und langsam 75 g Zinn-Granulat gegeben, und zur Vervollständigung der Reaktion wird die Mischung ca. 30 Minuten zum gelinden Sieden erhitzt. Nach dem Abkühlen wird die Reaktionslösung durch Zugabe von NaOH alkalisch gemacht, und durch Wasserdampfdestillation wird das Amin von der Reaktionslösung abgetrennt. Es befindet sich in den ersten 400 ml des Destillates. Das Destillat wird abermals mit NaOH versetzt und das Diamin scheidet sich als eine gelbliche, organische Phase ab. Diese wird abgetrennt und unter Argon-Atmosphäre bei Normaldruck destilliert.
- Ausbeute :: 0.082 mol (15.51 g) bzw. 82% d. Th.
weißer, hygroskopischer Feststoff
- Sdp.:: 142 - 143 °C
- ¹H-NMR:: -CH₃ = 1.12 ppm (s)
-NH₂ = 1.33 ppm (s)

### 2. Synthese des Bis-(5-nitrosalicyliden)-2.3-dimethyl-2.3-butandiyldiamin

7.929 g 2-Hydroxy-5-nitrobenzaldehyd werden in ca. 150 ml Ethanol suspendiert. Es resultiert eine gelb-orange Lösung mit Niederschlag, die zum gelinden Sieden erhitzt wird. Nach ca. 30 Minuten löst sich der Rückstand. Dann wird eine Lösung von 2.757 g 2.3-Diamino-2.3-dimethylbutan in ca. 30 ml Ethanol während 15 Minuten zur Reaktionslösung getropft. Mit Beginn der Zugabe fällt ein ockerfarbiger Niederschlag aus. Die Mischung wird 45 Minuten unter Rückfluß und ständigem Rühren erhitzt. Anschließend wird der Niederschlag heiß filtriert, mit heißem Ethanol gewaschen bis die Waschflüssigkeit farblos ist und im Exsikkator getrocknet.
- Ausbeute :: 9.12 g bzw. 93 % d. Th.
ockergelbes Pulver
- CHN-Analyse:: C : 58.05-57.82 (ber.: 57.97)
H: 5.41-5.45 (ber.: 5.35)
N : 13.41-13.50 (ber.: 13.52)

### 3. Synthese des N,N'-2.3-dimethyl2.3-butandiyl-bis(5-NO₂-salicyliden-iminato)cobalt(II), Co(5-NO₂-Saltmen)

4.144 g Bis-(5-nitrosalicyliden)-2.3-dimethyl-2.3-butandiyl-diamin werden in einem 3-Halskolben in 100 ml Ethanol suspendiert und unter Rühren und Erwärmen gelöst. In einem Erlenmeyerkolben werden 2.5 g Cobalt(II)acetat-tetrahydrat unter Erwärmen in 120 ml eines H₂O/EtOH-Gemisches (1:1) gelöst und langsam heiß während 60 Minuten zur Reaktionslösung getropft. Die Reaktionsmischung färbt sich orange und ein Niederschlag fällt aus. Die Mischung wird 2 Stunden unter Rückfluß und anschließend über Nacht bei Raumtemperatur gerührt. Dann wird die Reaktionsmischung erneut erwärmt und heiß filtriert, und der Niederschlag wird mit ca. 100 ml heißem EtOH bis zur Farblosigkeit der Waschflüssigkeit gewaschen. Der orange Rückstand wird im Vakuumexsikkator über P₂O₅ getrocknet.
- Ausbeute:: 4.37g bzw. 93% d. Th.
oranges Pulver

### 4. Aktivierung des N,N'-2.3-dimethyl2.3-butandiyl-bis(5-NO₂-salicyliden-iminato)cobalt(II)

In einem 250 ml 3-Halskolben mit Rückflußkühler werden 100 ml Pyridin unter Argon-Atmosphäre vorgelegt. Dann werden 4 g des Co(5-NO₂-Saltmen) in pulverisierter Form unter Rühren eingetragen, und die Mischung wird unter Rühren auf 120 °C erhitzt und 90 Minuten lang gerührt. Nach dem Abkühlen wird das aktivierte Co(5-NO₂-Saltmen) im Kühlschrank kristallisiert und filtriert. Der Rückstand wird bei 120 °C und 10⁻³ mbar etwa 1 Stunde getrocknet.

### Modifizierte Eintopfreaktion zur Synthese des N,N'-2.3-dimethyl2.3-butandiyl-bis(5-NO₂-salicyliden-iminato)cobalt(II), Co(5-NO₂-Saltmen)

- Ansatz:: 0.05 mol (12.45 g) Cobalt(II)-acetat*4 H₂O
0.1 mol (16.71 g) 2-Hydroxy-5-nitrobenzaldehyd
0.05 mol (9.46 g) 2.3-Diamino-2.3-dimethylbutan
0.2 mol (8.0 g) Natronlauge

In einem 500 ml 3-Halskolben gibt man 100 ml Methanol und 9.5 g 2.3-Diamino-2.3-dimethylbutan. Die Lösung wird unter Argonstrom zum beginnenden Sieden erhitzt. Dazu gibt man eine Lösung des Cobaltacetats in 120 ml heißem Wasser und schließlich eine entsprechende Menge festes NaOH. Die Farbe ändert sich von gelb nach rötlich. Jetzt wird der 2-Hydroxy-5-nitro-benzaldehyd gelöst in 100 ml Methanol zugetropft, wobei sich die Lösung intensiv rot färbt. Allmählich erfolgt die Bildung eines roten Niederschlages, der nach Vervollständigung der Reaktion (2 Stunden am Rückfluß) abgesaugt wird. Nach Waschen, Umkristalllisieren und Trocknen im Vakuum erhält man das tiefrote Produkt.
- Ausbeute:: 44.31 g bzw. 94% d. Th.
rotes Co(5-NO₂-Saltmen)
Im folgenden wird die Charakterisierung des Co(5-NO₂-Saltmen) beschrieben. Bei der Charakterisierung des Co(5-NO₂-Saltmen) bezogen auf das Sorptionsverhalten in Abhängigkeit des Sauerstoffpartialdruckes P(O₂) muß zwischen einer O₂-Sorption in Lösung und einer O₂-Sorption in festem Zustand unterschieden werden.

Die Bestimmung des reversiblen O₂-Sorptionvermögens der Carriermoleküle in Lösung wurde auf spektroskopischen Wege durchgeführt. Grundlage dieser Messung bildet die Tatsache, daß die reversible Anlagerung von Sauerstoff durch eine spektrale Änderung der Absorption von sichtbarem Licht begleitet wird. Sämtliche Messungen wurden unter Verwendung eines UV/VIS-Spektrophotometers der Fa. Perkin-Elmer Typ Lamda 15 durchgeführt. Die druckfesten Photometerküvetten wurden von der Fa. Hellma GmbH Typ "Flow-through Compact®" mit Suprasil I® Fenster bezogen.

Zur experimentellen Erstellung der Sorptionsisothermen wird eine eingestellte Lösung des Komplexes (etwa 10⁻³ molar) in einem koordinierenden Lösungsmittel wie DMSO oder in einem nicht koordinierenden Lösungsmittel wie Toluol oder Acetonitril unter Zusatz von 4-Dimethylaminopyridin in eine druckfeste Quarzküvette gefüllt. Diese Lösung wird unter Schutzgas in den Meßstrahl gebracht und gegen das reine Lösungsmittel im Vergleichsstrahl unter den selben äußeren Bedingungen photometrisch gemessen. Auf diese Weise lassen sich die Sorptionsbedingungen zwischen 100% Stickstoff (p_{O2} = 0 mbar), 20% (p_{O2} = 203 mbar), 50% (p_{O2} = 507 mbar), 80% (p_{O2} = 811 mbar) und 100% (p_{O2} = 1013 mbar) Sauerstoff beliebig variieren. Zusätzlich kann durch eine Druckbelastung der Küvette ein Sauerstoffpartialdruck bis etwa p_{O2} = 3000 mbar aufgebracht werden. Das Höhenverhältnis der Banden aus oxy- und deoxy-Form wird einer Auswertung zur Bestimmung der Sorptionskapazität zugrunde gelegt.

Zur Ermittlung der O₂-Sorption in fester Phase wurde ein gravimetrisches Verfahren gewählt. Die dazu aufgebaute Sorptionswaage beinhaltet als Kernstück eine Vakuum-Ultramikrowaage der Fa. Sartorius Typ 4102 mit elektronischer Anzeigeeinheit. Der Wägebereich beträgt maximal 3 g, die Wägegenauigkeit ±0.1 µg. Die gesamte Einheit läßt sich mit Hilfe eines Vacuubrand RD2 auf ein Endvakuum von etwa 10⁻⁴ mbar bringen.

Zur Messung wird der Komplex in einen Glastiegel am Meßarm der Balkenwaage eingewogen und mit einem entsprechenden normierten Gegengewicht ausgeglichen. Nun wird die Meßanordnung bei Raumtemperatur bis zur Gleichgewichtskonstanz evakuiert und die Meßänderungen mit Hilfe eines Meßwerterfassungsprogrammes registriert. Der dabei erhaltene Endwert wird als deoxy-Form definiert und bildet die Grundlage für weitere Sorptionsmessungen. Dazu wird nun die Waage mit Gasen der Zusammensetzung 100% Stickstoff (p_{O2} = 0 mbar), 20% (p_{O2} = 203 mbar), 50% (p_{O2} = 507 mbar), 80% (p_{O2} = 811 mbar) und 100% (p_{O2} = 1013 mbar) Sauerstoff belüftet und die auftretende Massezunahme als Funktion der Zeit registriert.

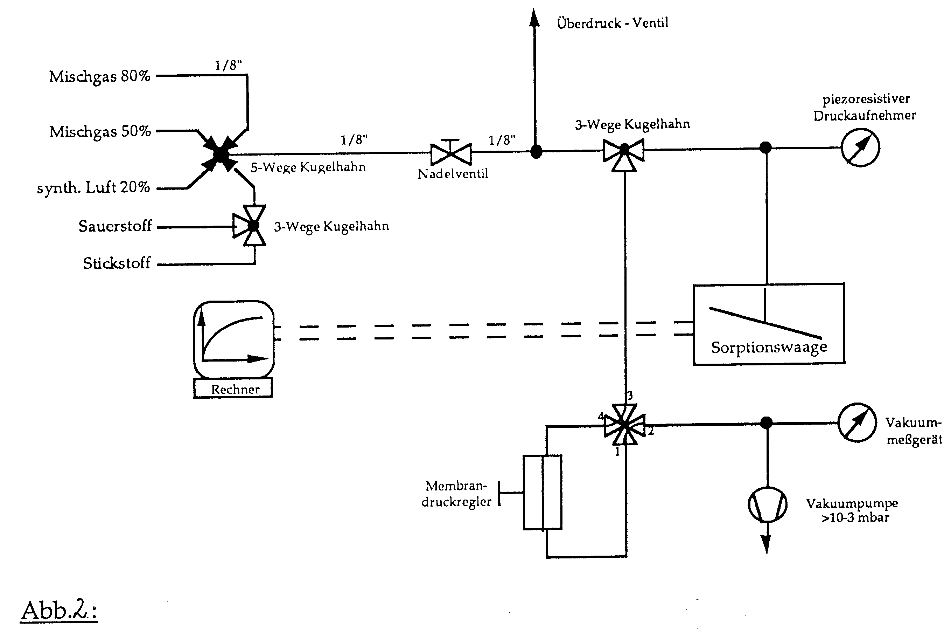

Abbildung 2 zeigt den Versuchsaufbau zur gravimetrischen Bestimmung der Sorptionsisothermen von Co(5-NO₂-Saltmen).

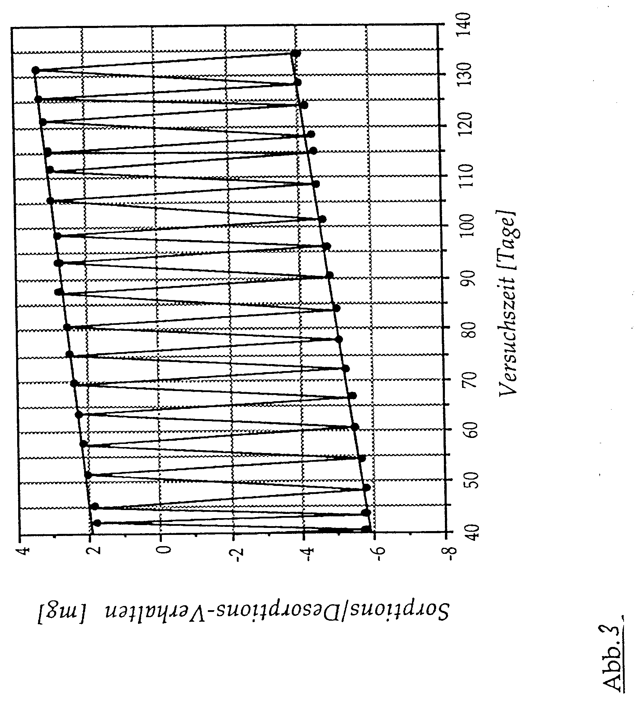

Abbildung 3 zeigt einen Ausschnitt aus dem Sorptions-Desorptionswechselverhalten von Co(5-NO₂-Saltmen). Der Anstieg resultiert aus der Waagen-Drift. Der Abstand beider Geraden bleibt konstant.

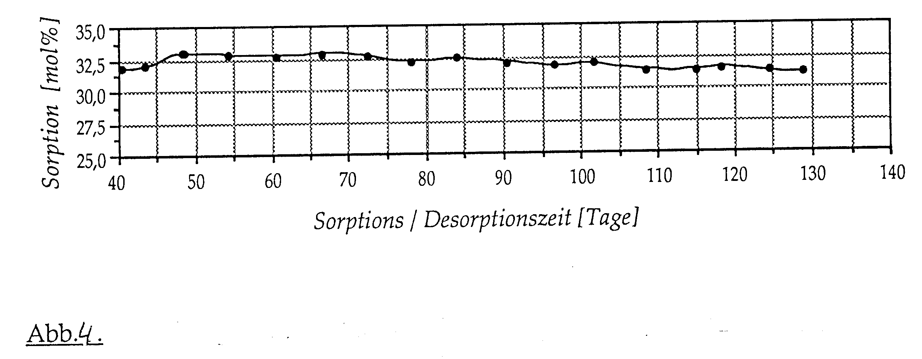

Abbildung 4 zeigt die Langzeitstabilität von Co(5-NO₂-Saltmen). Die ermittelte Zersetzungsrate ist kleiner als 0.014 Mol-% pro Tag.

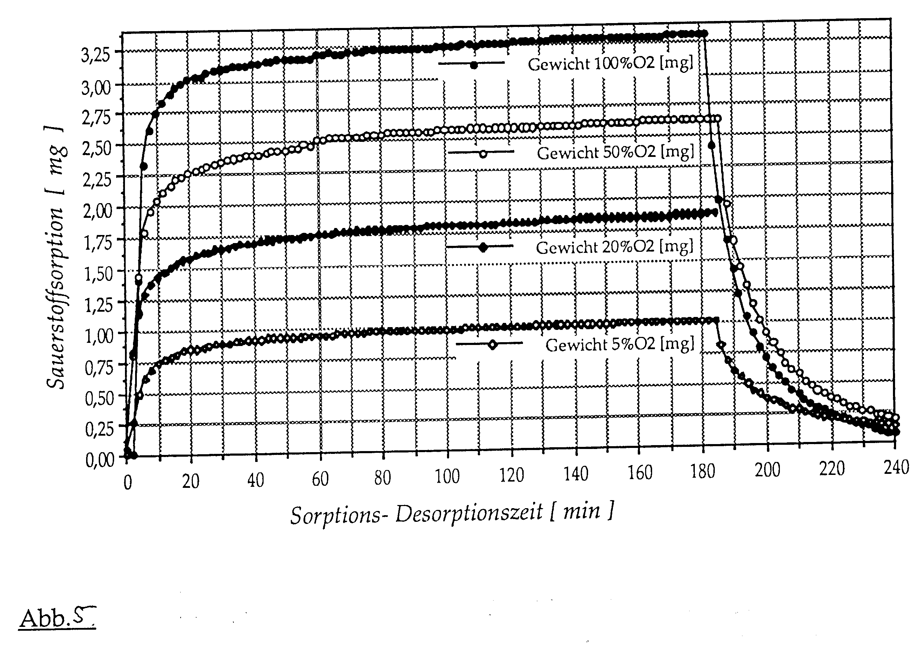

Abbildung 5 zeigt Sorptionsisothermen von Co(5-NO₂-Saltmen). Die Sättigungssorption in reinem Sauerstoff beträgt 36.3 Mol-%.

Aus diesen Untersuchungen geht hervor, daß die erfindungsgemäße Cobalt-Verbindung ganz hervorragend als Sauerstoff-Carrier geeignet ist. Sie kann in fester oder in gelöster Form und gegebenenfalls in Verbindung mit einer Axialbase als stoffspezifische Trägersubstanz (Carrier) für Sauerstoff verwendet werden, insbesondere zur selektiven Abtrennung von Sauerstoff aus Sauerstoff enthaltenden Gasen. Potentielle Anwendungen hierfür sind z.B. allgemeine Verbrennungsprozesse, denn durch die Anreicherung von Sauerstoff lassen sich hier höhere Flammtemperaturen, verbesserte ''Wärme-Ausbeuten'' und ein geringerer Brennstoff-Verbrauch erzielen. Weitere Anwendungen liegen bei der Kohleverflüssigung, bei der Herstellung von Peroxiden über einen erhöhten O₂-Partialdruck, bei Glas-und Metallschmelzverfahren, in der Abwasserbehandlung und in der Medizintechnik.

Die Anwendung kann z.B. so erfolgen, daß man ein Sauerstoff enthaltendes Gas mit einer sauerstoffdurchlässigen Membran in Kontakt bringt, die selektiv und reversibel Sauerstoff aus dem Sauerstoff enthaltenden Gas bindet und die die erfindungsgemäße Cobalt-Verbindung in fester oder gelöster Form enthält. Diese Membran trennt das Sauerstoff enthaltende Gas auf der einen Seite der Membran in einen Zulaufstrom und auf der anderen Seite der Membran in einen Produktstrom. Der Sauerstoff wird von der Produktstromseite der Membran desorbiert wird, wobei die Sauerstoffdesorption unter der Einwirkung eines Sauerstoffpartialdruck-und/oder eines Temperaturdifferentials das durch die Membran hindurch aufrechterhalten wird, durchgeführt wird. Bezüglich weiterer Ausführungen wird auf die EP 0098731 B1 und die EP 0098157 B1 verwiesen.

Desweiteren kann die erfindungsgemäße Cobalt-Verbindung gegebenenfalls in Verbindung mit einer Axialbase zur Herstellung einer Lösung verwendet werden, die ein reversibles sauerstoffkomplexbildendes Vermögen aufweist. Dabei fertigt man die Lösung aus einem Lösungsmittel oder einem Lösungsmittelgemisch, aus der erfindungsgemäßen Cobalt-Verbindung und aus einer Axialbase, wobei die Axialbase auch das Lösungsmittel oder das Lösungsmittelgemisch sein kann. Die Axialbase ist eine Verbindung, die zur Elektronenabgabe an das Cobalt-Atom der erfindungsgemäßen Cobalt-Verbindung befähigt ist. Bezüglich weiterer Ausführungen wird auch hier auf die EP 0098731 B1 und die EP 0098157 B1 verwiesen.

Desweiteren kann die erfindungsgemäße Cobalt-Verbindung, gegebenenfalls in Verbindung mit einer Axialbase, zur Herstellung von sauerstoffdurchlässigen, selektiv und reversibel Sauerstoff bindenden Membranen verwendet werden. Bezüglich der Herstellung solcher Membranen wird auf die folgenden Druckschriften verwiesen: EP 0098731 B1, EP 0176986 A2, EP 0186182 A2, EP 0213744 A2, US 3,625,734, US 4,705,544, US 4,174,374 und US 4,710,205.

Die erfindungsgemäßen Cobalt-Verbindungen sind bestens zur Herstellung von Flüssigmembranen, von Matrix-gestützten Flüssigmembranen oder von Fest-Carrier-Membranen geeignet.

Besonders gut sind die erfindungsgemäßen Cobalt-Verbindungen zur Herstellung von mikroinkapsulierten Flüssigmembranen nach einem Trocken-Naß-Phaseninversionsprozeß geeignet. Dabei fertigt man aus einer homogenen, flüssigen Polymerlösung, welche aus
a) 8 bis 35 Gew.-% eines organischen Polymers,
b) 20 bis 60 Gew.-% eines leichtflüchtigen Lösungsmittels,
c) 15 bis 80 Gew.-% eines schwerflüchtigen Lösungsmittels,
d) 1 bis 20 Gew.-% der erfindungsgemäßen Cobalt-Verbindung bzw. 10 bis 60 Gew.-% einer 0.02 bis 0.5 molaren Lösung der erfindungsgemäßen Cobalt-Verbindung in einem schwerflüchtigen, mit dem Fällungsmittel für die Phaseninversion nicht mischbaren Lösungsmittel, und gegebenenfalls aus
e) 5 bis 15 Gew.-% eines Nichtlösungsmittels (Fällmittel), besteht,
einen Polymerfilm, entfernt daraus das leichtflüchtige Lösungsmittel und unterzieht den resultierenden Polymerfilm mittels eines Fällmittels einer Phaseninversion.

Bei bevorzugten Ausführungsformen verwendet man eine Polymerlösung, die aus
a) 10 bis 20 Gew.-% eines organischen Polymers,
b) 40 bis 50 Gew.-% eines leichtflüchtigen Lösungsmittels,
c) 25 bis 35 Gew.-% eines schwerflüchtigen Lösungsmittels,
d) 2 bis 10Gew.-% der erfindungsgemäßen Cobalt-Verbindung und
e) 5 bis 10 Gew.-% eines Nichtlösungsmittels (Fällmittel)
oder aus
a) 10 bis 20 Gew.-% eines organischen Polymers,
b) 35 bis 45 Gew.-% eines leichtflüchtigen Lösungsmittels,
c) 25 bis 30Gew.-% eines schwerflüchtigen Lösungsmittels, und
d) 20 bis 40 Gew.-% einer 0.02 bis 0.5 molaren Lösung der erfindungsgemäßen Cobalt-Verbindung besteht.

Bevorzugte Polymere sind Polysulfone, Bisphenol-A Polysulfone, Polydimethylsiloxane, Polyethersulfone, Polyphenylsulfone, Polyimide, Polyetherimide, Polyphenylenoxide, Polycarbonate, Polyestercarbonate, Celluloseacetate, Polyurethane oder Polyetherblockamide.

Bevorzugte Axial-Basen sind 4-Dimethylaminopyridin, 1-Methylimidazol, 4-Cyanopyridin, Perfluortributylamin, Oligo(4-vinylpyridin) oder Oligo(1-vinylimidazol).

Bevorzugte leichtflüchtige Lösungsmittel sind Methylenchlorid, Chloroform, Aceton, Dioxan oder THF, bevorzugte schwerflüchtige Lösungsmittel sind NMP, DMAc, DMF oder DMSO, und bevorzugte Fällmittel sind Wasser, Ethanol, Methanol oder eine Mischung derselben.

Bevorzugt wird die Phaseninversion bei Temperaturen zwischen 15 und 35 °C durchgeführt.

Anhand von Ausführungsbeispielen wird die Verwendung der erfindungsgemäßen Cobalt-Verbindung zur Herstellung von Flüssigmembranen und zur Herstellung von Fest-Carrier-Membranen näher erläutert.

### Beispiel 1:

### Integral asymmetrische Fest-Carrier-Membran

- Rezeptur:: 100 g Dichlormethan
70 g N-Methylpyrrolidon
30 g Polysulfon
5 g Co(5-NO₂-Saltmen)·DMAP
≈ 8 g 1-Propanol
- Erzwungene Konvektion :: 20 Sekunden
- Freie Konvektion :: 15 Sekunden

### Durchführung:

Der Carrier Co(5-NO₂-Saltmen)·DMAP wird in 70 g N-Methylpyrrolidon in der Hitze unter Argon und in desorbiertem Zustand (Sauerstoff ist desorbiert) gelöst. Nun wird bei Raumtemperatur über 0.45 µm filtriert und die Lösung im Vakuum entgast. Das Polysulfon wird in Methylenchlorid gelöst, über 2 µm filtriert und im Vakuum entgast. In diese rotbraune Lösung wird nun unter langsamen Rühren 1-Propanol (5 - 7 ml) eingebracht, bis sich eine leichte Opaleszenz einstellt. Nach Rakeln auf Glas (200 µm) wird mit Stickstoff das leichtflüchtige Lösungsmittel abgeblasen, die Glasplatte unter Stickstoff kurz konditioniert und schließlich in Wasser koaguliert. Nach Ablösen von der Glasplatte wird kurz in Ethanol gespült und im Stickstoffstrom abgelüftet. Die Membran weist im statistischen Mittel eine Selektivität von S_{(O2/N2)} = 23 bei 30 °C und einen normalisierten Fluß von etwa 23 000 Ba/cm auf. Im REM ist ein sich nach oben verdichteter Schaum und eine porenfreie, dichte Haut an der Oberseite zu erkennen.

### Beispiel 2:

### Mikroinkapsulierte asymmetrische Flüssigmembran

- Rezeptur:: 100 g Dichlormethan
70 g N-Methylpyrrolidon
30 g Polysulfon
30 g Carrier-Lösung (.7m Co(5-NO₂-Saltmen)·DMAP in Diethylenglycoldibutylether)
- Erzwungene Konvektion:: 35 Sekunden
- Freie Konvektion :: 15 Sekunden

Durchführung: Der Carrier [Co(5-NO₂-Saltmen)·DMAP] wird in 30 g Diethylenglycoldibutylether und 30 g N-Methylpyrrolidon in der Hitze unter Argon und in desorbiertem Zustand gelöst. Nun wird bei Raumtemperatur über 0.45 µm filtriert und die Lösung im Vakuum entgast. Das Polysulfon wird in einem Gemisch aus Methylenchlorid und N-Methylpyrrolidon gelöst, über 2 µm filtriert und im Vakuum entgast. Nun werden die beiden Lösungen vermischt und gemeinsam mit Hilfe eines Ultraturax emulgiert. In diese tiefrote Lösung wird nun unter langsamen Rühren 1-Propanol (5 - 7 ml) eingebracht, bis sich eine leichte Opaleszenz einstellt. Nach Rakeln auf Glas (200 µm) wird mit Stickstoff das leichtflüchtige Lösungsmittel abgeblasen, die Glasplatte unter Stickstoff kurz konditioniert und schließlich in Wasser koaguliert. Nach Ablösen von der Glasplatte wird kurz in Ethanol gespült und im Stickstoffstrom abgelüftet.

| | | |
|---|---|---|
| Versuchsbedingungen: | Temperatur | 20 °C |
| | Feeddruck | 1.5 bar |
| | Permeatdruck | Atmosphärendruck (1 bar) |
| | Membranfläche | 63.62 cm² |
| | Stage-Cut | 0.1 |
| | Druckverhältnis | 0.14 |

Carrierhaltige Membranen zeigen nach Phaseninversion im Wasser eine reale Selektivität von S^{real} = 16.1. Da die reale Selektivität die eines carrierfreien asymmetrischen Polysulfonfilms deutlich überschreitet, wird ein trägergebundener Sauerstoff-Transport angenommen.

### Ergebnis :

| mikroinkapsulierte Flüssigmembran, Co(5-NO₂-Saltmen)/DEGBE | | | |
|---|---|---|---|
| Membran | Fluß O₂ [ml/min] | P/zO₂ [Ba/cm] | reale Selektivität |
| 1 | 1.71 | 40482 | 19.7 |
| 2 | 2.48 | 58711 | 13.2 |
| 3 | 2.22 | 52556 | 15.9 |
| 4 | 2.11 | 49952 | 15.6 |

| analoge, carrierfreie integral asymmetrische Gastrennmembran | | | |
|---|---|---|---|
| Membran | Fluß O₂ [ml/min] | P/zO₂ [Ba/cm] | reale Selektivität |
| 1 | 2.29 | 54212 | 6.58 |
| 2 | 2.13 | 50424 | 6.91 |
| 3 | 2.63 | 62261 | 6.27 |
| 4 | 1.99 | 47110 | 6.92 |

## Patentansprüche

1. N,N'-2.2-3.3-Tetramethylethylen-bis(5-NO₂-salicyliden-iminato)cobalt(II) der Formel

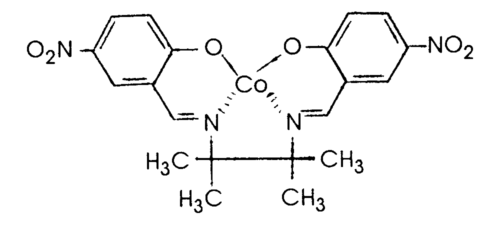

2. Verwendung der Cobalt-Verbindung nach Anspruch 1 in fester oder gelöster Form und gegebenenfalls in Verbindung mit einer Axialbase als stoffspezifische Trägersubstanz (Carrier) für Sauerstoff.

3. Verwendung nach Anspruch 2 zur Erzeugung von Sauerstoff oder zur selektiven Abtrennung von Sauerstoff aus Sauerstoff enthaltenden Gasen, **dadurch gekennzeichnet**, daß man ein Sauerstoff enthaltendes Gas mit einer sauerstoffdurchlässigen Membran, die die Cobalt-Verbindung nach Anspruch 1 in fester oder gelöster Form enthält, in Kontakt bringt, selektiv und reversibel Sauerstoff aus dem Sauerstoff enthaltenden Gas bindet, das Sauerstoff enthaltende Gas auf der einen Seite der Membran in einen Zulaufstrom und auf der anderen Seite der Membran in einen Produktstrom trennt und Sauerstoff von der Produktstromseite der Membran desorbiert wird, wobei die Sauerstoffdesorption unter der Einwirkung eines Sauerstoffpartialdruck- und/oder Temperaturdifferentials, das durch die Membran hindurch aufrechterhalten wird, durchgeführt wird.

4. Verwendung der Cobalt-Verbindung nach Anspruch 1 zur Herstellung einer Lösung, die ein reversibles sauerstoffkomplexbildendes Vermögen hat, gegebenenfalls in Verbindung mit einer Axialbase, **dadurch gekennzeichnet**, daß man die Lösung aus einem Lösungsmittel oder Lösungsmittelgemisch, aus der Cobalt-Verbindung nach Anspruch 1 und aus einer Axialbase herstellt, wobei die Axialbase auch das Lösungsmittel oder Lösungsmittelgemisch sein kann, und wobei die Axialbase eine Verbindung ist, die zur Elektronenabgabe an das Cobalt-Atom der Cobalt-Verbindung nach Anspruch 1 befähigt ist.

5. Verwendung der Cobalt-Verbindung nach Anspruch 1 zur Herstellung von sauerstoffdurchlässigen, selektiv und reversibel Sauerstoff bindenden Flüssigmembranen, Matrix-gestützten Flüssigmembranen oder Fest-Carrier-Membranen, gegebenenfalls in Verbindung mit einer Axialbase, **dadurch gekennzeichnet**, daß man aus einer homogenen, flüssigen Polymerlösung, welche aus
a) 8 bis 35 Gew.-% eines organischen Polymers,
b) 20 bis 60 Gew.-% eines leichtflüchtigen Lösungsmittels,
c) 15 bis 80 Gew.-% eines schwerflüchtigen Lösungsmittels,
d) 1 bis 20 Gew.-% der Cobalt-Verbindung nach Anspruch 1 bzw.
10 bis 60 Gew.-% einer 0.02 bis 0.5 molaren Lösung der Cobalt-Verbindung nach Anspruch 1 in einem schwerflüchtigen, mit dem Fällungsmittel für die Phaseninversion nicht mischbaren Lösungsmittel, und gegebenenfalls aus
e) 5 bis 15 Gew.-% eines Nichtlösungsmittels (Fällmittel), besteht,
einen Polymerfilm herstellt, daraus das leichtflüchtige Lösungsmittel entfernt und den resultierenden Polymerfilm mittels eines Fällmittels einer Phaseninversion unterzieht.

6. Verwendung nach Anspruch 5, **dadurch gekennzeichnet**, daß man eine Polymerlösung einsetzt, die aus
a) 10 bis 20 Gew.-% eines organischen Polymers,
b) 40 bis 50 Gew.-% eines leichtflüchtigen Lösungsmittels,
c) 25 bis 35 Gew.-% eines schwerflüchtigen Lösungsmittels,
d) 2 bis 10 Gew.-% der Cobalt-Verbindung nach Anspruch 1 und
e) 5 bis 10 Gew.-% eines Nichtlösungsmittels (Fällmittel) besteht.

7. Verwendung nach Anspruch 5, **dadurch gekennzeichnet**, daß man eine Polymerlösung einsetzt, die aus
a) 10 bis 20 Gew.-% eines organischen Polymers,
b) 35 bis 45 Gew.-% eines leichtflüchtigen Lösungsmittels,
c) 25 bis 30 Gew.-% eines schwerflüchtigen Lösungsmittels und
d) 20 bis 40 Gew.-% einer 0.02 bis 0.5 molaren Lösung der Cobalt-Verbindung nach Anspruch 1 besteht.

8. Verwendung nach Anspruch 5, 6 oder 7, **dadurch gekennzeichnet**, daß man als Polymer ein Polysulfon, Bisphenol-A Polysulfon, ein Polydimethylsiloxan, ein Polyethersulfon, ein Polyphenylsulfon, ein Polyimid, ein Polyetherimid, ein Polyphenylenoxid, ein Polycarbonat, ein Polyestercarbonat, ein Celluloseacetat, ein Polyurethan oder ein Polyetherblockamid einsetzt.

9. Verwendung nach einem oder mehreren der Ansprüche 5 bis 8, **dadurch gekennzeichnet**, daß man als Axial-Base 4-Dimethylaminopyridin, 1-Methylimidazol, 4-Cyanopyridin, Perfluortributylamin, Oligo(4-vinylpyridin) oder Oligo(1-vinylimidazol) einsetzt.

10. Verwendung nach einem oder mehreren der Ansprüche 5 bis 9, **dadurch gekennzeichnet**, daß man als leichtflüchtiges Lösungsmittel Methylenchlorid, Chloroform, Aceton, Dioxan oder THF einsetzt.

11. Verwendung nach einem oder mehreren der Ansprüche 5 bis 10, **dadurch gekennzeichnet**, daß man als schwerflüchtiges Lösungsmittel NMP, DMAc, DMF oder DMSO einsetzt.

12. Verwendung nach einem oder mehreren der Ansprüche 5 bis 11, **dadurch gekennzeichnet**, daß man als Fällmittel Wasser, Ethanol, Methanol oder eine Mischung derselben einsetzt.

13. Verwendung nach einem oder mehreren der Ansprüche 5 bis 12, **dadurch gekennzeichnet**, daß man die Phaseninversion bei Temperaturen zwischen 15 und 35 °C durchführt.
